# EUROPEAN PATENT APPLICATION

(11) **EP 4 534 110 A1**
(43) Date of publication of application: **09.04.2025**
(21) Application number: 23202250.9
(22) Date of filing: 06.10.2023
(51) Int. Cl.: A61L 2/00, A61B 50/00, A61B 90/70, A61L 2/04, A61L 2/12, B65B 55/02, H05B 6/64

(54) **TREATMENT OF MEDICAL MATERIAL BY MEANS OF MICROWAVES**

(71) Applicant: MEAM BV, 3530 Houthalen-Helchteren (BE)
(72) Inventor: Groffils, Carlo, 3020 Herent (BE); Groffils, Robert, 3840 Borgloon (BE); Van Geel, Renaat, 2880 Bornem (BE); de Meulemeester, Frédéric, 1130 Brussels (BE)
(74) Representative: De Clercq & Partners

(57) **Abstract**

The present disclosure relates to technology for treatment of a medical material. An aspect of the present disclosure relates to a method for treatment of a medical material, comprising the steps of: placing the medical material in a sealable container consisting of a sterilisation-resistant and non-microwave absorbent material; placing the container in a pressure vessel; applying microwaves to the container in the pressure vessel to heat and sterilise or decontaminate the medical material; and, applying overpressure to the container during microwave treatment to prevent deformation of the container.

## Description

### FIELD OF THE INVENTION

The present disclosure relates to technology for treatment of medical material. More specifically, the present disclosure describes a method and system for sterilizing or decontaminating medical material by applying microwaves to the medical material.

### BACKGROUND

Sterilising or decontaminating medical material such as infectious, contaminated or hazardous material from a healthcare facility, clinic or laboratory is a critical process that must be done properly to ensure the public safety. In particular, the disposal of liquid material, the material being of a medical origin or other origin, in high volumes is more challenging to handle compared to solid medical material and the transport and decontamination of liquid medical material may thus drastically increase the cost and make such a disposal thereof highly expensive, compared to the disposal of solid medical material.

The most commonly employed method for sterilization or decontamination is the use of a steam autoclave, which utilizes direct heat and steam to eliminate pathogens. However, steam autoclaves come with several noteworthy disadvantages that necessitate consideration. Firstly, they demand a substantial amount of energy for operation, resulting in elevated operational costs.

Furthermore, autoclaving can lead to the production of condensation, which has the potential to increase humidity levels within the sterilization chamber. While steam is intended to deliver heat to the load, the autoclave may experience condensation, which can hinder or even obstruct the passage of steam to the central regions of the load. When utilizing supersaturated steam, larger material lumps situated at the core of the load typically encounter a delay in temperature elevation, causing a disruption in the calculated time/temperature cycle. Additionally, in more stringent regulatory environments, the need to apply a vacuum at the end of the process is linked to reducing steam emissions into the atmosphere. Another drawback of steam autoclaves is their space requirements, which can be problematic for medical facilities with limited available space. These autoclaves employ either gravity or trolley systems for waste transfer. Additionally, the autoclave process can be time-consuming, involving extended sterilization cycles and cooldown periods. As a consequence, their operation can pose safety concerns, particularly when it comes to loading and unloading, as the high temperatures and pressures involved may pose burn or injury risks to personnel.

Accordingly, there is a need for methods and systems allowing a more efficient, cost-effective, and safe way of sterilising medical material, in particular liquid medical material. The present invention provides such methods and systems, which are described in the following patent application.

### SUMMARY OF THE INVENTION

It is an objective of the present invention to address the aforementioned needs by providing a more efficient, cost-effective, and safer method of sterilising medical material, in comparison to the existing treatment systems commonly used in healthcare environment facilities, clinics, or laboratories, such as steam autoclaves or other microwave-based systems.

Typically, the treatment of medical materials is performed inside a closed vessel, which often results in significant drawbacks. Specifically, this exposes the vessel walls to repeated cycles of high and low temperatures at a high moisture environment. These temperature cycles induce significant thermal stress on the vessel walls, leading to increased maintenance requirements. Consequently, downtime is necessary between each heating cycle to allow the vessel walls to cool down sufficiently for the safe handling of sterilized materials and preparation of the vessel for another batch, all while minimizing thermal stress. Therefore, another objective of the present invention is to improve the throughput of the sterilization process compared to existing systems.

To address these challenges, the present invention proposes a solution that achieves more localized heating of medical materials using microwaves (MW) generated within a pressurized vessel. Specifically, by placing the medical material in a container made of sterilization-resistant and non-microwave-absorbent material, heating efficiency can be improved, resulting in a faster and more energy-efficient sterilization protocol. Additionally, the locally heated air can enhance the heat distribution within the container, ensuring more uniform heating of the medical material. This is particularly important when dealing with stationary, non-moving targets, as microwave heating commonly leads to the formation of hot spots.

Furthermore, the container can act as a thermal barrier, preventing significant heating of the pressure vessel walls. This can significantly reduce the thermal stress exerted on the vessel walls, minimizing downtime and maintenance requirements. The latter is particularly important because of stringent regulatory requirements that necessitate frequent pressure vessel inspections to ensure safety standards. Consequently, higher throughput can be achieved since there's no need to wait for vessel cooldown between successive batches, and also allowing faster removal of the sterilised material and refilling the vessel with new medical material for sterilisation.

A first overview of various components according to aspects of the present invention is given hereinbelow, after which specific embodiments will be described in more detail. This first overview is meant to aid the reader in understanding the technological concepts more quickly, but it is not meant to identify the most important or essential features thereof, nor is it meant to limit the scope of the present invention. The invention is defined by the independent patent claims. The dependent claims define advantageous embodiments of the invention.

An aspect of the present disclosure relates to a method for the treatment, including sterilising or decontaminating, medical material using microwaves, said sterilisation method comprising the steps:
(a) placing the medical material in a sealable container made of a sterilisation-resistant and non-microwave absorbent material;
(b) placing the container in a pressure vessel;
(c) applying microwaves to the container in the pressure vessel to heat and sterilise the medical material which is contained in the container; and
(d) applying overpressure to the container during microwave sterilisation to prevent deformation of the container.

Another aspect of the present invention relates to a method wherein the heating rate is controlled to maintain a temperature within the container during microwave sterilisation that is suitable for sterilising the medical material, preferably wherein the temperature is at least 121°C.

In some embodiments, the heating rate is controlled to maintain the temperature within the container below the boiling point of water during microwave sterilisation, preferably below 138.9°C at 2.5 bara.

In some embodiments, the overpressure is between at least 1 bara and at most 3 bara, preferably between 1.5 bara and 2.5 bara, more preferably about 2 bara (bar above atmospheric pressure).

In some embodiments, the electric/magnetic field strength is between at least 1 kV/m and at most 7 kV/m, preferably between 1.5 kV/m and 5 kV/m, more preferably about 3 kV/m.

In some embodiments, the material of the container comprises polypropylene, preferably consists of polypropylene.

In some embodiments, the method comprises the step of measuring the temperature within the container during microwave sterilisation, and controlling the strength of the electric/magnetic field and thus the power of the microwaves applied to the container based on the measured temperature to maintain a desired temperature or temperature range within the container.

In some embodiments, the temperature is measured optically with an infrared sensor.

In some embodiments, the method comprises the step of measuring the pressure within the container during microwave sterilisation, and controlling the overpressure applied to the container based on the measured pressure to maintain a desired pressure or pressure range within the container.

In some embodiments, the method further comprises the step of placing the container in a transparent bag adapted to prevent water vapor generated during heating the medical material to escape the container.

In some embodiments, the microwaves are applied for a duration of between at least 10 minutes and at most 30 minutes, preferably between 15 minutes to 25 minutes.

In some embodiments, a sterilisation indicator means is disposed in the container for monitoring the efficacy of the sterilisation process. The sterilisation indicator may comprise a bioindicator comprising a sterilizable biological medium and a pH indicator to visually indicate that the biological medium is effectively sterilised.

In some embodiments, the method comprising the step of cooling the container with a cooling medium after microwave sterilisation, preferably by applying a cooling fluid or liquid to the container.

In some embodiments, the medical material comprises infectious or hazardous material from a medical facility, clinic and/or laboratory.

In some embodiments, the medical material is a liquid medical material.

In some embodiments, the medical material is a solid medical material, and wherein an amount of liquid is added into the container.

Another aspect of the present disclosure relates to a system for treatment of medical material, including sterilising or decontaminating of the medical material, the sterilisation system comprising:
(a) a container comprising a sterilisation-resistant and non-microwave absorbent material, and a lid forming a seal with the container; wherein the container is adapted to receive the medical material and optionally an amount of water;
(b) a pressure vessel adapted to receive the container and apply pressure to it;
(c) a microwave source adapted to generate microwaves in the pressure vessel; and
(d) a controller operatively connected to the pressure vessel and microwave source, wherein the controller is configured to apply microwaves to the container to heat and sterilise the medical material while applying overpressure to the container during microwave sterilisation to prevent deformation of the container.

In some embodiments, the system comprising a temperature sensor configured for measuring the temperature in the container and is in communication with the controller, wherein the controller is configured to control the power of the microwaves applied to the container based on the measured temperature to maintain a desired temperature or temperature range within the container.

In some embodiments, the pressure vessel of the system comprises a sight window, preferably formed in a wall of the pressure vessel, that allows visual inspection of the container; and an optical sensor preferably infrared sensor configured to measure the temperature of the container through the sight window during microwave sterilisation.

In some embodiments, the sight window of the system is adapted to prevent condensation , preferably with a heating means configured to warm the sight window.

Another aspect of the present disclosure relates to the system adapted for performing the method for sterilising of medical material.

Another aspect of the present disclosure relates to the use of the system for sterilising of medical material preferably comprising infectious or hazardous material from a medical facility, clinic and/or laboratory.

### DESCRIPTION OF THE FIGURES

The following description of the figures relate to specific embodiments of the disclosure which are merely exemplary in nature and not intended to limit the present teachings, their application or uses.

Throughout the drawings, the corresponding reference numerals indicate the following parts and features: treatment system (200) container (210); lid (220); bag (225); medical material (230); pressure vessel or autoclave (240); sight window of pressure vessel (245); microwave source (250); microwaves (255); controller (260); operative connection between the controller and the pressure vessel (270); operative connection between the controller and the microwave source (280); Infrared sensor (290); operative connection between the controller and the infrared sensor (295);
**Figure 1** is a block diagram (10) illustrating the steps of a method for treating medical material according to an aspect of the present invention.
**Figure 2** is a block diagram (100) illustrating the steps of a method for evaluating the treatment process using bioindicators according to an aspect of the present invention .
**Figure 3** is a schematic illustration of a treatment system (200) according to an aspect of the present invention.

### DETAILED DESCRIPTION

In the following detailed description, the technology underlying the present disclosure will be described by means of different aspects thereof. It will be readily understood that the aspects of the present disclosure, as generally described herein, and illustrated in the figures, can be arranged, substituted, combined, and designed in a wide variety of different configurations, all of which are explicitly contemplated and make part of this disclosure. This description is meant to aid the reader in understanding the technological concepts more easily, but it is not meant to limit the scope of the present disclosure, which is limited only by the claims.

The present invention pertains to the treatment of medical material, specifically the sterilisation or decontamination of the medical material, more particularly medical material containing a high content of liquid, which can be improved by employing a specific method. This involves placing the medical material in a container made of sterilisation-resistant material within a pressure vessel and applying microwaves (MW) to the container to heat and sterilise the medical material. Additionally, overpressure is applied to the container during microwave sterilisation to prevent deformation. This technology may significantly enhance the effectiveness and efficiency of sterilising medical material products with a high liquid content.

On a daily basis, medical facilities, clinics, and laboratories generate a large quantity of medical material, including both liquid and solid material, which must be sterilised or decontaminated before it can be handled, transported or reused. As used herein, "medical material" refers to a wide range of substances, objects, or products used in the field of medicine and healthcare. This can include materials that need to be sterilized before disposal, such as medical waste, or materials that need to be sterilized before they can be reused, such as medical equipment.

As used herein, "sterilization" refers to a process aimed at completely eliminating all forms of microbial life, including spores, bacteria, viruses, fungi, and bacterial spores, from a medical material. The end result of the sterilization process is a completely sterile medical material, where no viable microorganisms remain. Sterilization is relevant for medical materials where absolute microbial sterility is required, such as surgical instruments, pharmaceuticals, and certain laboratory equipment.

As used herein, "decontamination" refers to a process intended to reduce the microbial load to a safe and acceptable level, rather than achieving absolute sterility, from a medical material. The objective is to render a surface or object safe for handling or use without necessarily eliminating all microorganisms. Decontamination is relevant in general healthcare settings, such as cleaning and disinfecting common tools and utensils.

As used herein, "treatment" refers to both sterilization and decontamination of the medical material as defined above. While specific embodiments of the described technology may mention sterilization or decontamination individually, it is understood that the same embodiment can be applied for both sterilization and decontamination purposes unless otherwise specified.

The technology presented in the present invention can, therefore, be considered a 'generally applicable' sterilisation or decontamination technology, as it can be readily applied to a variety of materials, provided that they are suitable for sterilisation or decontamination using microwave energy in a contained and pressurised environment. The present invention can be regarded as equally suitable for sterilization or decontamination purposes but may require additional measures to achieve sterilization as the more rigorous process. For brevity, this technology will be described within the context of sterilization. However, all embodiments relevant to sterilization can be understood to be equally applicable for decontamination unless specified otherwise. The general applicability of this technology will be illustrated below with some exemplary applications.

In an exemplary application, the sterilisation of liquid medical material from a medical facility can be considered. This can include pharmaceutical material, such as expired or unused liquid medications and solutions, laboratory reagents, such as chemicals and liquids used in laboratory tests and experiments, various fluids used in medical or surgical procedures, bodily fluids from patient care, and similar items. Within this industry, the ease and efficiency of sterilisation are particularly crucial to improve safety and reduce overall transport costs. The technology's significant advantage lies in providing a sterilisation solution that requires little to no preparation, eliminating the need to add activation agents and is able to provide a high throughput of material to be sterilised. This allows for local sterilisation of the material without the addition of any extra products, which can greatly improve safety when handling medical liquid material and reduce costs associated with material transportation before or after sterilisation. As a result, the liquid-sterilised material no longer requires any special safety measures or specific transportation methods that would necessitate specialised and trained personnel.

In another exemplary application, the sterilisation of solid medical material from a medical facility can be considered. This can include used protective equipment worn by healthcare workers while treating patients, such as cleaning cloths, surgical gowns and plastic gloves, discarded or contaminated medical equipment, such as needles, scalpels, catheters and syringes, pharmaceutical packaging, laboratory material, soiled linens from the patient, and similar items. It is crucial to handle and manage solid medical material properly to prevent the spread of infections and protect healthcare workers and the public. Also, there is tendency for healthcare industry to move away from disposable use items to reusable items, necessitating the provision of alternative solutions for sterilising solid medical material. However, typical sterilisation processes often involve a significant amount of steam to reach the desired temperature and provide direct heat to the material for effective sterilisation and pathogen elimination within steam autoclaves.

Accordingly, sterilisation with MW under pressurised conditions is particularly advantageous since it allows for improved control of the heating parameters with faster response times if a temperature exceeds a specific threshold, as compared to difficult to control and slow to respond conventional sterilisation systems.

By employing the technology described in the present invention, the sterilisation process becomes more flexible and tailored to the specific type and volume of the medical material which needs sterilisation. This adaptability is for instance achieved by allowing the selection of different types and amounts of liquid to be added to the container holding the medical material. The choice of liquid depends on the characteristics of the solid material being sterilised. Different liquids require varying levels of microwave energy and pressure to achieve the high temperatures necessary for a successful sterilisation process. As an alternative to using a liquid to enhance the sterilisation process, solid microwave absorbents can be added to the solid medical material. These solid microwave absorbents are designed to absorb and dissipate microwave radiation effectively and will transfer the generated microwave radiation to the surrounding solid material to heat the material at or above the sterilisation temperature.

The present disclosure relates to methods and systems for sterilisation of medical material, which can allow for a more energy efficient and environmentally friendly sterilisation process of a variety of medical material products. This in turn can achieve a more economic sterilisation solution suitable for a wide range of local industrial applications. Specifically, the present system may reduce or altogether circumvent existing dangerous circumstances when handling and transporting contaminated medical or other material and requires little to no product preparation. This is particularly beneficial for batchwise sterilising because it allows for the safely preparing and handling of contaminated material by placing them in a container with lid and optionally placing the container inside a sealable bag prior to the sterilisation procedure, specifically compared to sterilisation systems that use steam to reach a specific operating temperature and as a result need the container holding the contaminated product to remain open and accessible by the steam during sterilisation. In case of the sterilisation of high-risk material, such as material contaminated with radioactive particles, a "zero risk bag in bag" procedure can be extremely beneficial. By placing the container holding the radioactive particles inside an additional and already sterilised sealable bag before placing the bag containing the container inside another bag before placing it into the pressure vessel, no radioactive particles can come into contact with the interior of the pressure vessel, reducing the risk of contamination, preventing extensive cleaning in between sterilisation cycles, and thus overall making the sterilisation of such contaminated materials a lot safer and cost efficient. As an alternative, especially if the container is intended for frequent reuse, the contaminated material can be placed inside an elastic container, such as a flexible bag, in which the contaminated material is collected from the different locations. This flexible bag can then be sealed and placed inside the rigid container, thus enhancing the safety when handling the contaminated material. Multiple flexible bags can be placed inside one or more rigid containers to increase the efficiency of the sterilisation process. Further, the present system may allow for improved monitoring of the sterilisation temperature and controlling of the sterilisation process such that it can be applied in a more efficient way, for example by allowing immediate termination when the threshold minimum sterilisation temperature of 121 °C is reached during a specific period of time, e.g. 30 minutes. To ensure that the pressure inside the container, and optionally inside the bag holding the container, is the same as the pressure inside the pressure vessel, a pressure control system having a pressure valve can be installed on the container. The pressure difference between the pressure inside the container and the pressure inside the pressure vessel can optionally be monitored by a controller configured to control the valve and adjust the pressure inside the container such that the pressures are advantageously equal or at least prevent significant deformation. As an alternative, an automatic pressure valve can be installed on the container configured to continuously regulate the pressure inside the container to match the pressure on the interior of the pressure vessel.

Further, using MW energy to heat the air inside the confinement of the container and monitoring of the sterilisation temperature within the container holding the contaminated material, allows for a more optimised heating and cooling rate process such that the throughput of the sterilisation process can be improved. The inventors have discovered that the utilisation of pressured air improves the efficiency of the sterilisation process. Pressurised air heated by MW energy improves the distribution of the heat, thus leading to a more uniform and homogeneous heating of the material compared to standard MW heating, where the material which needs heating is constantly turned around on a rotating platform to achieve a more evenly distribution of the heating and prevent the existence of cold and hot spots in the material when applying MW energy on the material. Using a container in a pressure vessel thus creating a smaller enclosure compared to the entire pressure vessel and applying MW energy to the pressurised air in the confined space of the container, to primarily heat the pressurised air inside the container only, can provide a homogeneous heating of the interior of the container and material contained in the container, while the temperature inside the pressure vessel can remain lower, thereby maintaining cooler vessel walls that reduces the thermal stress exerted on the pressure vessel. The reduction of the thermal stress on the walls of the pressure vessel can decrease maintenance requirements and provide for reduced down-time of the pressure vessel. Additionally, the throughput of the sterilisation process is increased since the waiting time for the vessel cooldown between batches is reduced.

Additionally, the improved energy efficiency can be combined with various "green" energy sources to achieve more an eco-friendlier sterilisation solution. Specifically, the present system can be more easily integrated and synchronised into smaller electric grid, preferably smart grids providing energy obtained from solar and wind power. To clarify, because of the reduced start-up times, the sterilisation can be synchronised with the green energy generation, e.g., as soon as the environmental conditions allow for generation of wind and/or solar energy the drying can be initiated to operate during high energy peaks (low costs) and avoid low energy peak (high cost).

Unless otherwise defined, all terms used in describing the technology, including technical and scientific terms, have the meaning as commonly understood by one of ordinary skill in the art to which this disclosure belongs. By means of further guidance, definitions for the terms used in the description are included to better appreciate the teaching of the present disclosure. The terms or definitions used herein are provided solely to aid in the understanding of the technology.

As used herein, "moisture content" refers to the amount of water contained in the product which is commonly expressed as a percentage of the weight of a product.

As used herein, a "liquid material" refers to a product to be sterilised, whereby the product is primarily in a liquid form. The liquid material product may have a moisture content of at least 50 %, preferably of at least 55 %, more preferably of at least 60 %, more preferably still of at least 65 %, more preferably still at least 80 %, more preferably still at least 90 %, more preferably still at least 95 %, more preferably still at least 99 % or more. The skilled person understands that the moisture content of a provided liquid material product depends on the type of material or product. However, for a lower moisture content, such as 75 % or less, it may be advantageous to include a liquid such as a solvent to increase the moisture content by dissolving the solid components present in the liquid material, before proceeding with the sterilising process. The liquid may include water or another solvent. For example, other liquids commonly found in medical materials can include various alcohols (e.g. ethanol and isopropyl alcohol), Formaldehyde (e.g. in formalin solutions), saline solution, antiseptic solutions, hydrogen peroxide, gels, blood products, media, dyes, stains, and so on. These liquid may create vapours but as long as these are sterilised or decontaminated, they can be safely eliminated.

As used herein, a "solid material" refers to a product to be sterilised, whereby the product is in a primarily solid form. The solid material product may have a moisture content of less than 50 %. The skilled person understands that the moisture content of a provided liquid material product depends on the type of material or product. However, for a moisture content below 50 % or lower it may be advantageous to include a liquid such as a solvent to dissolve solid components, or to increase the overall liquid available in the container by saturating the solid material such as fabrics before proceeding with the sterilising process. The liquid may include water or another solvent. As an alternative, solid microwave absorbents can be added to the solid material to increase the efficiency of the sterilisation process. Adding such solid microwave absorbents may be combined with the addition of a liquid.

As used herein, a "decontaminated product" is a product, either solid or liquid, that has undergone a sterilisation process for a minimum time, for example, 30 minutes, at a minimum temperature, such as 121°C. This sterilisation process may effectively decontaminate the product, rendering it safe to be handled and transported without the risk of further contamination when in contact with the product. The skilled person understands that the required sterilisation time and the minimum and maximum temperatures, or the boiling temperature, of the product inside the container may vary depending on the type of medical material and its moisture content.

As used herein, "humidity" refers to the concentration of water vapour present in the air, which is commonly expressed as a relative humidity based on temperature and water content. When using a closed container, the vapor present in the air when heating and sterilising is primarily contained inside the container. When a container and lid are used, whereby the lid has an opening to allow temperature measurement inside the container, it may be convenient to place the container and lid in a flexible bag adapted to prevent vapor from escaping the flexible bag and from entering inside the sterilisation chamber of the pressure vessel.

A minimum humidity of 10%, preferably 20% with a maximum of 50% is desirable, while excessive humidity above 50% can be advantageously avoided, since this may increase the energy requirements to reach the temperature suitable for sterilisation. Additionally, high humidity may cause excessive condensation on the colder walls of the pressure vessel or on the see-through window of the pressure vessel. Nonetheless, the medical material can be drained prior to sterilization to reduce the humidity, provided that the liquid can be safely drained.

As used herein, "microwave (MW) sterilisation" concerns the sterilisation of a product by means of microwave technology as is known in the art. This technology relies on the ability of microwaves to generate heat rapidly within the target material, which leads to the destruction of harmful microorganisms, such as bacteria, viruses, and fungi.

During MW sterilisation, the preferred sterilisation temperature of the material or product which needs sterilisation largely depends on the balance between the energy generated by the water dipoles in the microwave field and the energy absorbed by water molecules evaporated from the surface of the material. In an exemplary embodiment MW sterilisation can be employed by generating microwaves having an adjustable electric/magnetic field strength up to 6 kV/m and more preferably between at least 1 kV/m and at most 7 kV/m, preferably between 1.5 kV/m and 5 kV/m, preferably about 3 kV/m. In order to improve the energy efficiency of the sterilisation process, it is possible to have a first heating cycle whereby the product temperature is raised as fast as possible above the minimum temperature, whereby, as soon as the minimum temperature is reached, a second heating cycle is operated such that the product temperature is maintained between the minimum sterilisation temperature and the maximum boiling temperature of the product which needs sterilisation.

As an example, during the first heating cycle, electric/magnetic field strength between 3kV/m and 7kV/m is used until the product temperature is above the minimum sterilisation temperature of e.g. 121°C at 2.5 bara and below the boiling temperature e.g. 138.9°C at 2.5 bara (i.e., 2.5 bar above atmospheric pressure), and preferably between 1kV/m and 3kV/m during a second heating cycle, where the product temperature is maintained around the preferred sterilisation temperature (e.g. 121°C) of the material or product. For 138°C at 3.5 bara, 10 minutes can be enough to achieve a log 8 (Microbial inactivation = 10⁸) sterilisation. Nonetheless, the person skilled in the art understands that various other electric/magnetic field strength can be employed and the herein described microwave sterilisation is not limited to a specific electric/magnetic field strength thereof or the use of one or more heating cycles.

The "preferred sterilisation temperature" is the ideal temperature for a particular product set between the minimum sterilisation temperature of 121°C, which is required to kill any pathogens, and the boiling temperature of the particular product.

The person skilled in the art understands that, depending on the industrial application of the sterilisation process and depending on the type of product to be sterilised, different configuration can be employed to sterilise the product by adjusting the electric/magnetic field strength, pressure inside the sterilisation cavity of the autoclave, the size and shape of the container and lid, and/or the bag in which the container is placed. Further, depending on the product, a different minimum duration of the sterilisation process may be necessary, as well as different heating cycles and/or a different ideal temperature to maintain throughout the entire sterilisation process. Exemplary embodiments of such configurations will be described throughout the present disclosure.

An overview of various aspects of the technology of the present disclosure is given hereinbelow, after which specific embodiments will be described in more detail. This overview is meant to aid the reader in understanding the technological concepts more quickly, but it is not meant to identify the most important or essential features thereof, nor is it meant to limit the scope of the present disclosure, which is limited only by the claims. When describing specific embodiments, reference is made to the accompanying drawings, which are provided solely to aid in the understanding of the described embodiment.

An aspect of the present disclosure relates to a method for sterilising of medical material such as liquid material or solid material, said sterilisation method comprising the steps:
(a) placing the medical material and optionally an amount of microwave-safe liquid and/or solid microwave absorbents in a sealable container comprising of a sterilisation-resistant and non-microwave absorbent material;
(b) placing the container in a pressure vessel;
(c) applying microwaves to the container in the pressure vessel to heat and sterilise the medical material placed in the container; and
(d) applying overpressure to the container during microwave sterilisation to prevent deformation of the container.

Compared to traditional steam autoclaving methods for sterilising products such as medical material, the present invention offers several advantages. Firstly, microwave sterilisation can be performed in a fraction of the time required for steam autoclaving, reducing downtime, and improving efficiency. This is because the utilisation of air pressure improves the sterilisation process efficiency. The pressurised air inside the container and heated by specifically directed microwaves, improves the distribution of heat, leading to a more uniform and homogenous heating of the medical waste inside the container. Additionally, the use of microwaves can result in lower energy consumption compared to steam autoclaving, resulting in reduced operating costs. Additionally, the container can act as a barrier to prevent the heating of the walls of the pressure vessel. If the temperature of the vessel walls is kept as low as possible, the overall time to heat and cool the medical material according to the sterilisation process is reduced, since only a small enclosure holding the medical waste needs to be heated and cooled.

The present invention therefore provides a more efficient, cost-effective, and safe method for sterilising medical material compared to traditional steam autoclaving methods.

In some embodiment, the heating rate can be controlled to maintain a temperature within the container during microwave sterilisation that is suitable for sterilising the medical material, wherein the temperature is preferably at least 121 °C.

In some embodiments, the heating rate can be controlled to maintain the temperature within the container below the boiling point of water during microwave sterilisation, preferably below 138.9°C at 2.5 bara.

In some embodiments, the overpressure can be between at least 1 bara and at most 3 bara, preferably between 1.5 bara and 2.5 bara, more preferably about 2 bara.

Applying overpressure to the container during the heating process has the advantage of preventing deformation of the container which can occur due to the heating of the material of the container itself above its deformation point or when the pressure and heat are not dissipated equally, and which can potentially compromise the sterilisation process. The specific pressure range may be determined based on factors such as the material and thickness of the container, the size and composition of the medical material being sterilised, and the heating and cooling rates used in the process. In general, the pressure range should be sufficient to prevent deformation of the container but not so high that it poses a safety risk or creates unnecessary complexity in the sterilisation system. A pressure range between 1 and 2 bara can be used for sterilisation of medical material.

It is important to note that while the pressure range of 1.5 to 2.5 atmospheres (atm) or bara has been exemplified, the specific pressure range may vary depending on the sterilization system's design and the requirements of the medical materials to be sterilized. In particular, the temperature and time parameters can influence the overall cycle duration. To achieve higher temperatures, it may be possible to increase the pressure, a relationship that can be balanced by adjusting the temperature accordingly. Notably, if the load is fully sterilized due to favourable time and temperature parameters, the vessel can be easily purged with air to expedite load cooling, as pressure can and should be applied at the start of the process. In some embodiments, the electric/magnetic field strength can be between at least 1 kV/m and at most 7kV/m, preferably between 1.5 kV/m and 5 kV/m, more preferably about 3 kV/m.

As described herein, the material of the container can comprise, and preferably consist of, a sterilization-resistant material. This means that the container material can withstand the sterilization process without undergoing significant physical or chemical changes, such as degradation, melting, becoming porous, or otherwise losing its structural integrity or functional properties when exposed to the sterilization conditions.

Furthermore, the material of the container can include, and preferably consist of, a non-microwave-absorbent material. This means that the container material can allow microwaves to pass through without significant absorption. It is advantageous for the material to be dielectrically inert, meaning it does not interact with microwave radiation. It is to be understood that, while it is advantageous, it is not required for the entire container to consist of a non-microwave-absorbent material. For example, the container may comprise components such as valves or seals, as described in further embodiments, that may consist of partially or fully microwave-absorbent material, or one or more walls of the container may consist of partially or fully microwave-absorbent material, as long as the container when considered as a unit, enables sufficient microwaves to pass through to allow sterilization of the contained medical material.

In an embodiment, the material of the container can comprise plastic, glass, ceramic or a combination thereof. There are various materials known in the art that are sterilisation-resistant and non-microwave absorbent to microwaves such that the medical material which is contained inside the container can be sterilised without having energy wasted on heating and sterilising the container itself.

In an embodiment, the material of the container can comprise a plastic material, preferably polypropylene. In a preferred embodiment the container can consist of a plastic material, preferably polypropylene. Such a polypropylene container is sterilisation-resistant and non-microwave absorbent to microwaves such that the medical material which is contained inside the container can be sterilised without having energy wasted on heating and sterilising the container itself.

Another advantage of the present invention is that the use of a closed container within the pressure vessel can help to contain any hazardous materials or odours produced before and/or during sterilisation. This can help to improve the safety of personnel working in the vicinity of the sterilisation equipment.

When a closed container is used, it may be important to monitor the pressure inside the pressure vessel and the closed container. The difference between the pressure in the pressure vessel and the container should be kept to zero. If not, the container, whether or not the container is inside a bag, can deform, implode or explode, depending on the pressure differences. Therefore, a pressure control system may be included, for example a valve installed on the container in case of a closed container, or installed within the closed bag in case of using a bag to seal the container, which is configured to (automatically) regulate the differences in pressure between the interior and exterior of the closed container. The valve can be a two-way valve configured to automatically regulate the variation in pressure. As an alternative, a controllable pressure valve and a control system can be added whereby the pressure is measured by the pressure control system inside the pressure vessel and inside the container or bag. Depending on the difference in pressure, the valve can be controlled to lower the difference in pressure, for example back to zero. As a result, the pressure control system and overpressure applied to the container can help to prevent any deformation or damage to the container during sterilisation, ensuring that the medical material is properly contained and allowing for the container to be re-used after a sterilisation routine. In addition, the use of a sterilisation-resistant and non-microwave absorbent material can help to reduce material volume and disposal costs, as certain types of medical material that cannot be effectively sterilised by steam autoclaving can be safely sterilised using the present invention. Typically, the sterilisation-resistant and rigid container can be re-used after the sterilisation process of the medical material. Especially if the medical material is already directly packaged inside an elastic container, such as inside an flexible bag, after usage and at the location where it was used and disposed of, the rigid container itself can be re-used conveniently while avoiding direct contact with the contaminated medical material.

In an embodiment, the method may comprise the step of measuring the temperature within the container during microwave sterilisation and controlling the power of the microwaves applied to the container based on the measured temperature to maintain a desired temperature or temperature range within the container. Since an overpressure is applied to the container during microwave sterilisation, it is preferred that the temperature measurements are performed through a window installed in a wall of the pressure vessel and whereby the temperature sensor is placed outside the pressure vessel and measuring through the window.

In particular, the system can include a sight window and an infrared sensor, allowing operators to monitor the sterilisation process in real-time and ensuring that the temperature of the closed container remains below the boiling point of the liquid inside the medical material. However, a temperature sensor may be installed inside the container as well whereby a signal is sent to a controller for monitoring the temperature inside the container.

While microwaves are being applied to the container within the pressure vessel, the liquid particles of the product can undergo heating, causing the temperature inside the container to exceed the minimum sterilization temperature. This temperature elevation results from the consistent and uninterrupted supply of microwave energy to the product enclosed within the container.

Furthermore, to mitigate the risk of container deformation, it is possible to introduce overpressure within the interior of the pressure vessel. This measure significantly enhances the container's ability to retain its structural integrity when subjected to overpressure within the pressure vessel. It is important to note that minor deformations may occur naturally, particularly during repeated cycles or high applied pressure. Nevertheless, it remains advantageous for precise process control that the container maintains its form, minimizing the potential for harm to, or distortion of, the medical materials stored within the container. Additionally, the application of overpressure can result in an elevation of the temperature inside the container, which is placed within the pressure vessel. This increase in temperature can be advantageous for the sterilization process. To achieve sterilization, it is advantageous to sustain a temperature within the container above a minimum threshold of 121°C, while preventing the product from reaching a boiling point. Therefore, the introduction of overpressure within the pressure vessel serves to facilitate the rise in fluid temperature inside the product without allowing it to reach its boiling point, a scenario that could occur in the absence of overpressure.

In an embodiment, the method may comprise the step of measuring the pressure within the container during microwave sterilisation, and controlling the overpressure applied to the container based on the measured pressure to maintain a desired pressure or pressure range within the container. Since the pressure is measured, an accurate control of the sterilisation process by using a control system is possible. In an embodiment, the sterilisation process can be controlled by the control system on the basis of at least one of the following four parameters. The first parameter being the incident power, measured at the MW source, the second parameter being the absorbed power, which is calculated by the control system, thirdly, the actual temperature of the product inside the container which is measured e.g. by an IR temperature sensor through the sight window of the pressure vessel, and lastly, the applied pressure present inside the vessel, and thus also inside the container.

As a result, reducing or increasing the pressure can regulate the temperature increase or decrease inside the container in a more rapid manner. Therefore, the preferred operating temperature, being a temperature between a minimum and maximum temperature, can be maintained more easily. Also, when the temperature is too high, so reaching close to the boiling point of the product inside the container, it is possible that the container can start to deform, which may permanently damage the container, and which may cause the contaminated product to spill from the container and leak inside the pressure vessel. Adjusting the pressure inside the pressure vessel can prevent such deformation.

The control system can be configured to adapt the sterilisation process by measuring and/or calculating at least one of the above-described parameters and implementing changes to the sterilisation process. These changes may include an increase or decrease of the applied MW power, or an increase or decrease of the applied pressure. Further, an optical sensor may be present configured to monitor any deformation of the container due to an inappropriate pressure inside the pressure vessel. The signals of the optical sensor may be sent to the control system to identify if a deformation occurs and to make the necessary changes to the sterilisation process to prevent further deformation.

In an embodiment, the method comprises the step of placing the container in a flexible bag adapted to hermetically seal the container within. Upon opening the pressure vessel's door, the contents of the vessel may cease to maintain sterility, assuming the successful completion of the sterilization process. This can be due to the contamination introduced by the microbiological air environment. Therefore, the medical material can be advantageously hermetically sealed prior to treatment. This sealed external enclosure ensures that post-treatment, the sterilized materials remain protected from contamination. Alternatively or in combination, the medical material can be placed in a hermetically sealed flexible bag that is placed into the container.

In an embodiment, the method comprises the step of placing the container in a flexible bag adapted to prevent water condensation from occurring on the inside walls of the pressure vessel. Advantageously the bag is transparent, allowing visibility of the materials on the inside. Placing the container inside such a transparent bag, e.g. a thin plastic bag consisting of LDPE, will have a number of advantages. Although a lid may be foreseen which is adapted to close of the container, the lid may not be airtight, and water vapor may still be able to evacuate from openings between the closed off container and lid. When placing the closed container inside the bag, it is now possible to maintain the evaporated water inside the closed bag and prevent the humidity to increase to a point where water vapor may condense on the inside and colder walls of the pressure vessel. This is particularly important and helpful when a temperature sensor, such as an IR temperature sensor is used which is placed outside the pressure vessel and which can measure the temperature inside the container through a sight window of the pressure vessel.

To prevent condensation from occurring on the sight window, it is important to limit the humidity and thus the amount of water vapor being present inside the pressure vessel. However, in case the humidity level inside the pressure vessel is too high due to any reason, the excess liquid needs to be drained to decrease the humidity level inside and to prevent condensation on the interior walls. By doing so, it can significantly decrease the cleaning time needed between two batches of medical material which needs sterilisation since the inside walls of the pressure vessel will need minimal cleaning.

Further, in case of using an infrared (IR) temperature sensor, an opening may need to be foreseen inside the lid of the container, since this would increase the accuracy of the measurements of the IR temperature sensor. The IR temperature sensor can determine the temperature inside the container through the sight window and through the opening inside the lid. However, having such an opening in the lid can allow evaporation of the generated steam from the container. If no transparent bag were used, this generated steam would be able to pass freely inside the pressure vessel and could potentially condensate on the sight window, thereby blocking the clear view of the IR temperature sensor on the interior of the container and thus affecting the temperature measurements. An additional advantage of using a transparent bag is that the space surrounding the container is kept to a minimum thus making the area which needs to be heated up smaller and faster to reach the minimum required temperature for sterilisation.

Although an IR temperature sensor is typically preferred to measure temperature, other sensors known in the art such as thermocouple, thermistor, fibre optic sensor, may be used to replace the IR temperature sensor, or may be used together with the IR temperature sensor for redundancy. Nonetheless, since the IR temperature sensor allows remote sensing of temperature, it provides the advantage of not being exposed to the pressurised environment, which may damage or lead to erroneous readings in the other types of sensors.

In an embodiment, condensation on the interior of a sight window can be prevented by pre-heating the window with hot air before starting the magnetron. Alternatively, heating wires can be embedded in the window and the window can be heated using these heating wires.

In an embodiment, the microwaves can be applied for a duration of between at least 10 minutes and at most 30 minutes, preferably between 15 minutes to 25 minutes. The theoretically required sterilisation time of water is 30 minutes at 121°C with an applied overpressure of 2.5 bara. However, depending on the type of liquid present in the material which needs sterilisation, a different minimum duration to achieve adequate sterilisation may be possible.

In another embodiment, the microwaves can be applied for a duration below 10 minutes, for example 2 or 3 minutes, to achieve a very fast cycle. This can be more suitable for decontamination purpose although sterilisation can still be applied provided that the Temperature can be raised sufficiently high during that time period.

In an embodiment, a sterilisation indicator means can be disposed in the container for monitoring the efficacy of the sterilisation process. The sterilisation indicator may comprise a bioindicator comprising a sterilisable biological medium and a pH indicator to visually indicate that the biological medium is effectively sterilised. In order to determine if the sterilisation process was successful, it is necessary to determine if an adequate temperature and dwell time were applied. This can be evaluated by using sterilisation indicators, such as Sterikon^{®} plus Bioindicators which are commonly used to control the sterilising process in autoclaves. These Sterikon^{®} plus Bioindicators are designed for sterilisation processes at 121°C. Before revealing the result, which is a change in colour, the indicators need to be incubated at 60°C for at least 24 hours. If the sterilisation has not been complete and thus unsuccessful, the microbes in the liquid of the ampuls start to grow and cause a change in the colour. In this particular indicator, the liquid will turn from pink to yellow. If however the sterilisation was successful, the colour will not change since the microbes in the liquid of the ampuls were destroyed and thus unable to grow and change the colour. In an embodiment, at least one, but preferably three or more ampuls are placed at various locations inside the liquid. To exclude the effects of post-handling and incubation conditions, each test batch may contain an untreated, that is non-sterilised but incubated, ampul as reference to be compared with the ampuls which are placed inside the liquid, and which are sterilised together with the medical material. As an alternative, especially if the medical material is already pre-packaged inside an flexible bag, the bio-indicators can be placed in between the stacked flexible bags to check and indicate if the appropriate sterilisation levels are reached.

In an embodiment, the method may comprise the step of cooling the container with a cooling medium after microwave sterilisation, preferably by applying a cooling fluid such as gas or a liquid to the container and/or the interior of the pressure vessel. Alternatively, a heat exchanger may be used together with the application of cooling medium or without such a cooling medium in order to reduce the cooling time of the container. For smaller pressure vessels, peltier elements or thermoelectric coolers can be considered. A further alternative may be to isolate the surface of the pressure vessel by applying an isolating layer along the vessel surface to minimise the heat exchange to the vessel walls in the first place. Reducing the time needed to cool the container and its content allows for a quicker overall sterilisation cycle and a safer handling of the batches when taking out of the pressure vessel after sterilisation.

In an embodiment, typically the medical material comprises infectious or hazardous material from a medical facility, clinic and/or laboratory. The medical material may be a liquid medical material or a solid medical material where an amount of liquid is added into the container prior for sterilisation.

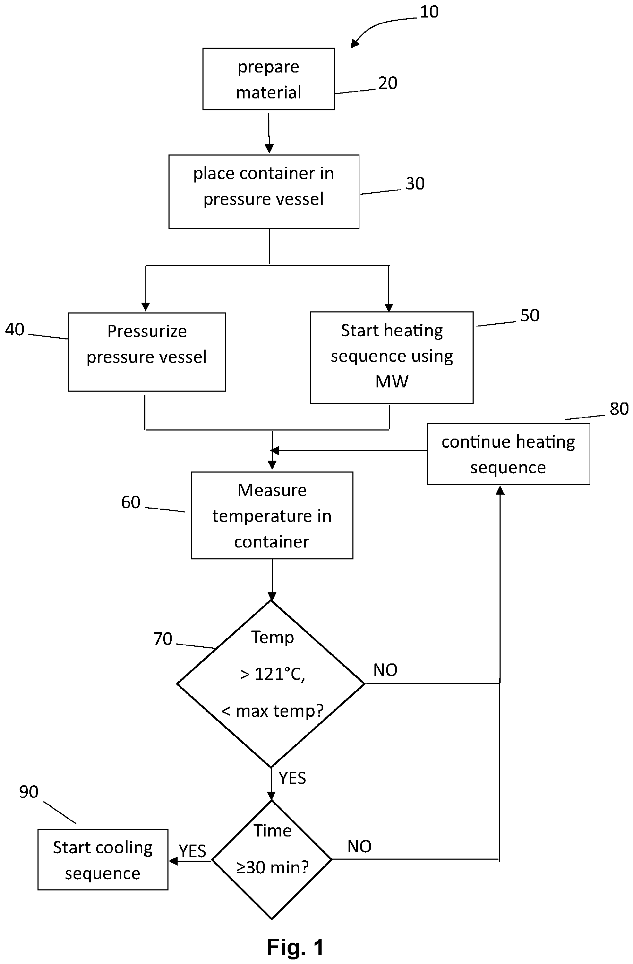

The present sterilisation method 10 will be discussed in more detail with reference to Figure 1, which is a block diagram illustrating the steps of said method according to a general embodiment.

In step 20, the method may include the step of preparing the container containing the medical material to be sterilised. If the medical material is a liquid, no further liquid such as water needs to be added to the medical material, since the medical material in itself will be able to heat due to the microwave energy which will be applied. However, depending on the type of liquid medical material, it may still be advisable to add water to the container in order to speed up the heating and thus sterilisation process. If water, or another liquid is added to the container, may be possible to add the water under the liquid medical material or to place it in a separate spacing dedicated for receiving such additional water. In case the medical material is a solid medical material, it may be necessary to add liquid to the solid material in order to saturate the solid material such as surgical cloths, or to dissolve the solid material such that the solid material becomes a semi or complete liquid material.

In step 30, the method may include placing the medical material inside the container. After placement, the container can be left open, or it can be closed by a lid to prevent easy access to the medical material during further handling and avoid spillage. When using a lid, the medical material which is contained within the container will be housed in a smaller environment, compared to when the container is left open and placed as is inside the pressure vessel or autoclave. By having a closed container, the medical material inside the container can heat up much faster and reach the minimum temperature required for sterilisation, which is 121°C.

In step 60, the method may include the step to measure the temperature of the medical material inside the container. In an embodiment, this step may comprise placing of a temperature sensor inside the container, or advantageously, inside the medical material contained inside the container. Such a temperature sensor is able to directly measure the core temperature of the medical material. The signal generated by the sensor can be send to a controller for evaluation and monitoring. A temperature sensor which is able to resist under pressure is to be preferred since the pressure vessel will be placed under pressure.

Alternatively, an IR temperature sensor can be placed outside the pressure vessel and will measure the core temperature of the medical material through a sight window which is foreseen in the pressure vessel or conclave. In order for the IR temperature sensor to work in a proper and correct way, it may be necessary to provide an opening in the lid of the container, such that an unhindered view is achieved by the IR temperature sensor on the medical material inside the container which will provide for a more accurate reading of the temperature inside the container.

Further, the container with the lid having an opening may be placed inside a thin bag, e.g. a plastic bag made of LDPE material. Such a thin plastic bag will prevent the evaporation of the steam generated by the medical material inside the interior of the pressure vessel when heating up the waste by the MW energy. This may be especially beneficial when the steam would otherwise condensate on the glass sight window of the pressure vessel which would potentially block the view of the IR temperature sensor towards the medical material inside the container.

In step 70, the method may include a sterilisation after closing of the vessel. In an exemplary embodiment, during an initial phase, an overpressure 40 of e.g. 2.5 bara, (which corresponds to a 3.5 bar absolute compared to space) can be applied. Water boils at 138,9°C at 2.5 bara overpressure, which is sufficiently above the minimum target temperature of 121°C required for sterilisation. By doing so, the temperature measured by the temperature sensor and the time necessary to control and adjust the MW energy can occur without the liquid, e.g. water inside the container to reach the temperature of 138.9°C which is the boiling point of water at 2.5 bara overpressure.

Simultaneously or subsequent to the applying of the overpressure 40, the heating sequence 50 can start. Therefore, electric/magnetic field strength of about 4 kV/m is directed to the container holding the medical material until the product temperature is between 121°C and 138.9°C. Once this operating temperature is reached, the field strength can optionally be reduced to 1-3 kV/m to maintain the operating temperature at the desired level. Reducing the power can allow for a more energy friendly sterilisation process 10.

During the heating sequence, the temperature is constantly measured 60 and evaluated by a controller. The controller can determine 70 if the temperature is above 121°C and below the maximum set boiling temperature. If the core temperature of the product is below 121°C, the electric/magnetic field strength needs to remain at the same level in order to continue the heating sequence 80. As soon as the core temperature is equal to or above 121°C, the electric/magnetic field strength may be reduced in order to save energy while the heating sequence may continue. As soon as the core temperature is above the set minimum temperature of 121°C, a timer can start to run. If the time that the core temperature is above 121°C is less than the minimum required time, e.g. 30 minutes, the heating sequence can continue 80. However, if the time that the core temperature is above 121°C is equal to or above the minimum required time, the cooling sequence 90 may optionally be started in order to cool down the core temperature of the medical material such that the pressure vessel can be opened again and the container holding the medical material can be handled in a safe manner.

In the simplest manner, the cooling sequence 90 entails the powering down of the applied electric/magnetic field strength to zero, and allowing the medical material inside the container to cool down. Once cooled down sufficiently, the overpressure is released from the pressure vessel. However, it is also possible to reduce the cooling down time by applying liquid gas inside the pressure vessel to more rapidly cool down the interior of the pressure vessel. When the container is placed inside the plastic bag, it is possible to spray cold water inside the interior of the pressure vessel and onto the plastic bag holding the container in order to cool down the temperature of the container and thus the content of the container. Another alternative could be the use of a heat exchanger in order to reduce the cooling time. An advantage of using such a heat exchanger is that the heat generated by the heat exchanger can be used for different purposes.

Once the core temperature of the material is sufficiently low, e.g. 100°C, preferably 60°C or less, the pressure vessel can be opened safely.

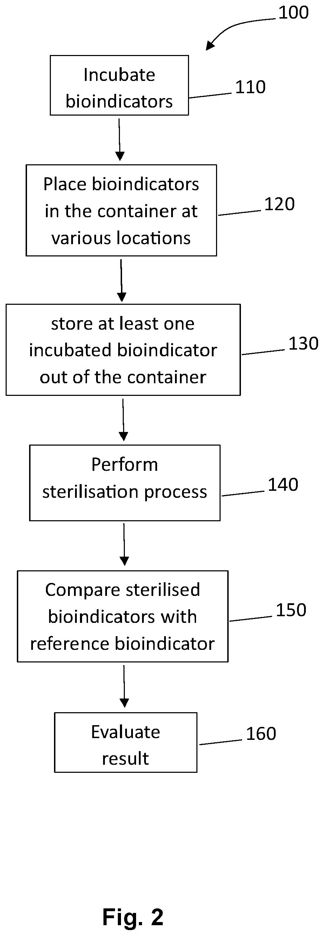

In order to determine if the overall sterilisation cycle was sufficiently long, a check can be done using a visual marker. For example, **Figure 2** is a block diagram 100 illustrating the steps needed to prepare, use and evaluate the sterilisation process using a bioindicator as a marker for a successful sterilisation cycle. A commonly used bioindicator is Sterikon ^{®} Plus bioindicator, which is designed for sterilisation processes at 121°C. Other bioindicators are also known in the art which can be used with other temperatures used at sterilisation processes. In order to prepare the bioindicators such that they can be used during the sterilisation process, an incubation phase is needed 110. Once incubated, e.g. at 60°C during at least 24 hours, they can indicate if a sterilisation process is done correctly by changing colour if the sterilisation process was incorrect or by an absence of a change in colour, by remaining at the same colour after incubation. If the sterilisation cycle was not completed, the microbes in the liquid of the ampuls will start to grow and cause a change in the colour. The liquid inside the bioindicators will then turn from pink to yellow, while in case of an adequate sterilisation, the liquid would remain its pink colour. To check the efficiency of the sterilisation process, i.e. if adequate temperature and dwell time were applied, e.g. three ampuls of Sterikon ^{®} Plus bioindicators are placed at various locations in the medical material 120 and will undergo the same sterilisation cycle as the medical material. At least one incubated ampul is kept outside the pressure vessel 130 and can serve as a reference ampul when later checking the e.g. three ampuls which were inside the pressure vessel. After the sterilisation cycle 140, the three ampuls are compared 150 with the reference ampul. Evaluation of the sterilisation process is done by comparing the colours of the reference ampul with the sterilised ampuls 160. When only the reference ampul changes colour from pink to yellow, while the remaining three ampuls maintain their pink colour, the sterilisation cycle was successful. However, if all or some of the ampuls also change colour from pink to yellow, the sterilisation cycle was not successful and needs to be repeated for this particular batch of medical material in order to be certain that an appropriate level of sterilisation was accomplished.

If the reference ampul does not change colour from pink to yellow, the incubation process of the ampuls was not successful and the result provided by the ampuls cannot be relied upon.

Although Sterikon ^{®} Plus bioindicators are commonly used, different types of bioindicators may be better suited when a higher or lower sterilisation temperature is required.

Building upon the above embodiment, an automated read-out of a visual marker, such as a bioindicator, can be envisioned using a camera or another type of optical unit configured to automatically read the optical signal provided by the visual marker. For example, an algorithm can be implemented to automatically detect the colour or range of colours of the visual marker and compare it to a baseline colour to categorize a sterilisation cycle as successful or failed. Other variations of this aspect can be implemented in accordance with the knowledge of those skilled in the art."

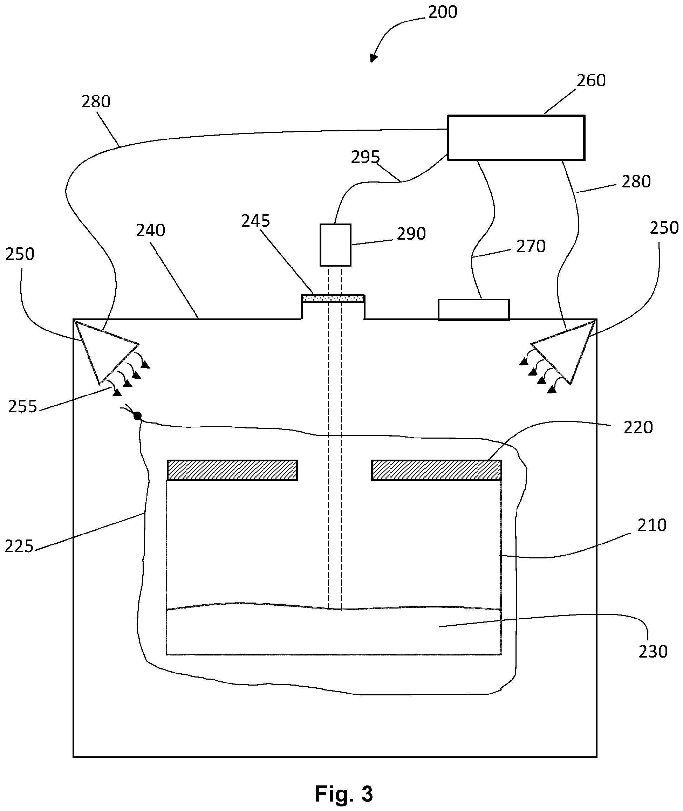

Another aspect of the present disclosure relates to a system 200 for sterilizing of medical material which will be discussed in detail with reference to **Figure 3**, which is a schematic illustration of the sterilisation system 200. Said sterilizing system 200 comprising:
(a) a container 210 comprising a sterilisation-resistant and non-microwave absorbent material, and a lid 220 forming a seal with the container 210; wherein the container 210 is adapted to receive the medical material 230 and *optionally* an amount of liquid such as water;
(b) a pressure vessel 240 adapted to receive the container 210 and apply pressure to it;
(c) a microwave source 250 adapted to generate microwaves in the pressure vessel 240; and
(d) a controller 260 operatively connected 270 to the pressure vessel 240 and operatively connected 280 to the microwave source 250, wherein the controller 260 is configured to apply microwaves 255 to the container 210 to heat and sterilise the medical material 230 while applying overpressure to the container 210 during microwave sterilisation to prevent deformation.

The container 210 is preferably comprised of a sterilisation-resistant and non-microwave absorbent polymer material such as polypropylene, glass, ceramic or a combination thereof. The skilled person will understand that the material of the container 210 may comprise some amount of microwave absorbent materials but the general properties of the container need to be such that it allows microwaves to pass through without significant absorption in order to heat up the medical material 230 placed in the container.

Alternatively, the container 210 may have a sufficiently large opening at the top of the container 210 which will allow the microwaves 255 to heat up the medical material 230 from the top only. A lid 210 may be foreseen which can be placed in the opening of the container 210, such that the container 210 can be closed to allow safe handling of the container 210 e.g. when placing inside the pressure vessel 240 before sterilisation.

Additionally, it may be possible to place the container 210, with or without lid 220, inside an, optionally transparent, bag 225 that can be closed such that the steam generated when heating the material 230 inside the container 210 is contained inside the transparent bag 225 and thereby cannot escape from the container to the interior of the pressure vessel 240. By preventing the steam from escaping, condensation on the glass of the sight window 245 can be greatly reduced and any incorrect measuring of the temperature is avoided. In order to make sure that the pressure inside the container 210 and optionally inside the transparent bag 225 holding the container 210 is the same as the pressure inside the pressure vessel 240, a pressure control system (not shown) having a pressure valve can be installed on the container 210. The pressure difference between the pressure inside the container 210 and the pressure inside the pressure vessel 240 can optionally be monitored by a controller 260 which can control the valve and to regulate the pressure inside the container 210, bag 225 or pressure vessel 240, such that both pressures are equal. As an alternative, an automatic pressure valve is installed on the container 210 or transparent bag 225, which is able to constantly regulate the pressure inside the container 210 or bag 225 with the interior of the pressure vessel 240.

The microwave source 250 can be installed inside the pressure vessel or autoclave 240, embedded in the walls of the pressure vessel or autoclave 240, or outside the pressure vessel or autoclave 240. When the microwave source 250 is placed outside the pressure vessel 240, it is important that the microwaves 255 are able to penetrate the walls of the pressure vessel 240 in order to heat up the medical material 230 inside the container 210. Although one microwave source 250 may be sufficient to heat up the medical material 230 which is placed inside the container 210, it may be beneficial to have more than one microwave source 250 since this may speed up the heating of the medical material 230 to the minimum required temperature required for sterilisation.

The microwave source can comprise a generator that is commercially available on the market. Preferably, microwave source can comprise a solid-state microwave generator. In some embodiments, the solid-state microwave generator can operate with a power between 25 W and 2000 W, preferably between 50 W and 1500 W, more preferably between 75 W and 1000 W, more preferably still between 100 W and 500 W, for example, between 50 W and 450 W, or between 75 W and 350 W, or between 100 W and 325 W, or between 125 W and 300 W, or between 150 W and 275 W, or between 175 W and 250 W, or between 175 W and 225 W, for example 200 W. Combinations of power amplifiers can be used to reach the desired operating power; for example, to supply a 200 W semiconductor generator with power, two 100 W amplifiers can be used, or four 50 W amplifiers, etc.

In an embodiment, the generated microwaves can have a frequency of at least 0.1 to at most 6.0 Ghz, for example, 0.2 GHz, 0.3 GHz, 0.4 GHz, 0.5 GHz, 0.6 GHz, 0.7 GHz, 0.8 GHz or 0.9 GHz; more preferably at least 1.0 GHz, for example 1.1 GHz, 1.2 GHz, 1.3 GHz, 1.4 GHz, 1.5 GHz, 1.6 GHz, 1.7 GHz, 1.8 GHz or 1.9 GHz; more preferably at least 2.0 GHz, for example 2.1 GHz, 2.2 GHz, 2.3 GHz, 2.4 GHz, 2.5 GHz, 2.6 GHz, 2.7 GHz, 2.8 GHz or 2.9 GHz; more preferably still at least 3.0 GHz, for example 3.1 GHz, 3.2 GHz, 3.3 GHz, 3.4 GHz, 3.5 GHz, 3.6 GHz, 3.7 GHz, 3.8 GHz or 3.9 GHz; more preferably still at least 4.0 GHz, for example 4.1 GHz, 4.2 GHz, 4.3 GHz, 4.4 GHz, 4.5 GHz, 4.6 GHz, 4.7 GHz, 4.8 GHz or 4.9 GHz; more preferably still at least 5.0 GHz, for example 5.1 GHz, 5.2 GHz, 5.3 GHz, 5.4 GHz, 5.5 GHz, 5.6 GHz, 5.7 GHz, 5.8 GHz or 5.9 GHz.

In any of the aforementioned embodiments, it is evident that the sterilization or decontamination criteria are intricately tied to the applied temperature and the process parameters. To attain elevated temperatures, it may be feasible to increase the pressure, and this relationship can be balanced by making corresponding adjustments to the process time. For example, when the vessel or container is heavily loaded, it may be advantageous to increase the microwave power to achieve higher temperatures more rapidly, or alternatively, extending the overall processing time to ensure thorough sterilization or decontamination. The same applies for variations in the properties of the medical material, such as its humidity and content.

In an embodiment, the pressure vessel 240 can comprises a sight window 245, preferably formed in a wall of the pressure vessel 240, that allows visual inspection of the container 210; and an optical sensor 290 preferably infrared (IR) sensor 290 configured to measure the temperature of the container 210 through the sight window 245 during microwave sterilisation. Condensation may be present due to the heating of the medical material 230. As the medical material 230 is heated, steam may be generated which may escape out of the container 210.

The sight window 245 may therefore be adapted to prevent condensation, preferably with a heating means configured to warm the sight window 245. In order to heat up the sight window 245 before or during the sterilisation cycle, hot air may be directed to the sight window 245 of the pressure vessel 240. Such hot air stream can be applied to the inside of the sight window 245 when the pressure vessel 240 is still open and being prepared for the upcoming sterilisation process. Alternatively, means to provide a stream of hot air which is directed to the inside of the sight window 245 may be provided, which can provide a hot air stream when the pressure vessel 240 is already closed and in operation. Another alternative may be to provide electrical heating wirers inside the sight window 245 such that the sight window 245 may be heated up before or during the sterilisation cycle. A skilled person will appreciate that a combination of these heating means is also possible. When the glass of the sight window 245 is heated up, the presence of condensation on this window can be reduced to a minimum, thus maintaining a clear view for the IR temperature sensor 290 on the medical material 230 inside the container 210. Further, it is possible to perform a temperature measurement prior to the sterilisation process to determine any deviation due to the glass surface of the sight window 245 and to check the accuracy of the IR temperature sensor 290 which can be used. Therefore, the temperature of a reference liquid is measured directly in the liquid inside the interior of the pressure vessel 240, e.g. by using a PT100 temperature sensor, and simultaneously through the glass of the sight window 245 using the IR temperature sensor 290. If a transparent bag is present during the sterilisation process, this bag can also be placed around the container to mimic the actual setup which can be used during the sterilisation process itself. These reference measurements can be done after pre-heating of the glass to further minimise the error when performing the actual temperature measurements with the IR temperature sensor 290 during the sterilisation process. The measured temperatures are compared, and the deviation can be stored in the controller 260 for calibration purposes and are used to adjust the temperature measurements of the IR temperature sensor during operation to provide a more accurate temperature reading and thus control of the sterilisation process.

Further to receiving the calibration value, the controller 260 is able to receive data 295 from the IR temperature sensor 290 during the sterilisation process. Further, pressure data can be received 270 from the pressure vessel 240 and the controller 260 is further able to control the pressure vessel 240 based on the temperature reading of the IR temperature sensor 290. Likewise, the controller 260 is able to control 280 the one or more microwave sources 250 depending on the temperature reading of the IR temperature sensor 290 and/or of the received pressure data. During the sterilisation process, the pressure vessel 240 is pressurized up to e.g. 2.5 bara and once this preset pressure was reached, a field strength of e.g. 4 kV/m is applied. Although the preferred manner is to apply the appropriate pressure first before applying microwaves, it is possible to simultaneously apply the pressure and the microwaves. During a first heating cycle, the medical material 230 inside the container 210 is heated as fast as possible such that the minimum temperature for sterilisation is reached in an as short amount of time as possible. Although preferred, a more gradual increase of the temperature may be required and in such a situation, a step-by-step increase of the electric/magnetic field strength may be more appropriate to reach the minimum temperature for sterilisation.

Typically, the minimum temperature required for sterilisation is 121°C during a time period of 30 minutes according to industry guidelines and regulations. Such a combination of temperature and time is known as "autoclaving" and is widely recognized as an effective method of sterilisation for medical material. By setting a temperature of at least 121°C, the claims are ensuring that the sterilisation process is effective in killing all microorganisms, including bacteria, viruses, and fungi, that may be present in the medical material. Typically, the boiling temperature of the material will correspond to 138.9°C, which is the boiling temperature of water at 2.5 bara. However, different boiling temperatures may be possible and a different minimum sterilisation temperature and/or maximum boiling temperature may be set by an operator prior to the start of the sterilisation process and stored in the controller 260. Likewise, a different pressure may be set by the operator prior to the start of the sterilisation process. Consequently, these settings may vary depending on the properties of medical material 230, such as the quantity, the power consumption and speed, the overall time required for the sterilisation process, etc.

Once the temperature measured by the IR temperature sensor 290 is above 121°C, a second heating cycle may start whereby the temperature of the material 230 inside the container 210 is kept above the minimum required temperature of e.g. 121°C but below the boiling temperature of the material. This is done by reducing the applied electric/magnetic field strength to e.g. 3 kV/m to maintain the temperature in the appropriate temperature range of 121°C and 138.9°C. Depending on the temperature reading by the IR temperature sensor 290, the electric/magnetic field strength can be reduced further or increased again by the controller 260 to maintain the material temperature in the appropriate temperature range. Once the second heating cycle is started, a timer can be configured to monitor the total duration where the temperature was above the minimum required sterilisation temperature, and below the boiling temperature. On regular time intervals, the IR temperature sensor can measure the temperature of the material 230 inside the container 210 and can provide this temperature reading to the controller 260. The controller 260 can then check if the temperature is in the appropriate temperature range, and can verify how much time has already passed since material temperature was in the appropriate temperature range. If the material temperature was in the appropriate range during at least e.g. 30 minutes, the second heating cycle can be terminated, and a cooling sequence may start. If the set time duration of e.g. 30 minutes was not yet reached, the second heating cycle continues, and the material temperature is maintained between the minimum and maximum required temperature by a continued application of electric/magnetic field strength.

As explained before, the cooling of the interior of the pressure vessel 240 may be done in various manners and once the temperature of the material 230 reaches the set cooling temperature, the sterilisation process is finished and the pressure vessel 240 may be opened and the container holding the sterilised material may be removed from the pressure vessel. As an alternative, or in addition to the cooling of the interior walls of the pressure vessel 240, an isolation layer to reduce the heating of these walls may be applied to the interior of the pressure vessel 240. Although cooling down of the interior walls of the pressure vessel may still be required, the cooling time may be significantly reduced due to such an isolation layer. Typically, the cooling temperature of the material 230 is about 100°, but a different temperature may be selected by the operator.

When bioindicators were placed inside the container 210 together with the material 230 before and during the sterilisation process, these bioindicators can now be safely removed from the medical material 230 inside the container 210 and the sterilised bioindicators can now be compared with the reference incubated bioindicator to determine if the sterilisation process was successful.

Different shapes of container 210 are possible and the shape and size of the container 210 used may vary depending on the type of containers which are readily available in the market. Typically, the interior of a pressure vessel 240 is cylindrical. As the cavity is cylindrical, to maximize the volume the container 210 may hold while still be insertable inside the interior of the pressure vessel 240, a cylindrical reservoir or container 210 may be preferable. To allow the IR temperature sensor to measure the temperature of the material 230 inside the cylindrical container 210, an opening is needed in the outer circumference of the cylindrical container 210. This opening can conveniently be used to insert the material such as liquid material 230 inside the cylindrical container 210. If a cylindrical container 210 is used, it may be necessary to foresee means configured to keep the cylinder 210 in an upright position such that the opening is facing the sight window 245 of the pressure vessel 240 at all times.

The described system 200 can be used to sterilise medical material comprising infectious or hazardous material from a medical facility, clinic and/or laboratory.

Reference throughout this specification to "one embodiment" or "an embodiment" means that a particular feature, structure or characteristic described in connection with the embodiment is included in at least one embodiment of the present disclosure. Thus, appearances of the phrases "in one embodiment" or "in an embodiment" in various places throughout this specification are not necessarily all referring to the same embodiment.

As used herein, the terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps. The singular forms "a", "an", and "the" include both singular and plural referents unless the context clearly dictates otherwise.

As used herein, relative terms, such as "left," "right," "front," "back," "top," "bottom," "over," "under," etc., are used for descriptive purposes and not necessarily for describing permanent relative positions. It is to be understood that such terms are interchangeable under appropriate circumstances and that the embodiment as described herein are capable of operation in other orientations than those illustrated or described herein unless the context clearly dictates otherwise.

Objects described herein as being "adjacent" to each other reflect a functional relationship between the described objects, that is, the term indicates the described objects must be adjacent in a way to perform a designated function which may be a direct (*i.e.* physical) or indirect (*i.e.* close to or near) contact, as appropriate for the context in which the phrase is used.

Objects described herein as being "connected" or "coupled" reflect a functional relationship between the described objects, that is, the terms indicate the described objects must be connected in a way to perform a designated function which may be a direct or indirect connection in an electrical or nonelectrical (*i.e*. physical) manner, as appropriate for the context in which the term is used.

As used herein, the term "substantially" refers to the complete or nearly complete extent or degree of an action, characteristic, property, state, structure, item, or result. For example, an object that is "substantially" enclosed would mean that the object is either completely enclosed or nearly completely enclosed. The exact allowable degree of deviation from absolute completeness may in some cases depend on the specific context. However, generally speaking the nearness of completion will be so as to have the same overall result as if absolute and total completion were obtained. The use of "substantially" is equally applicable when used in a negative connotation to refer to the complete or near complete lack of an action, characteristic, property, state, structure, item, or result.

As used herein, the term "about" is used to provide flexibility to a numerical value or range endpoint by providing that a given value may be "a little above" or "a little below" said value or endpoint, depending on the specific context. Unless otherwise stated, use of the term "about" in accordance with a specific number or numerical range should also be understood to provide support for such numerical terms or range without the term "about". For example, the recitation of "about 30" should be construed as not only providing support for values a little above and a little below 30, but also for the actual numerical value of 30 as well.

The recitation of numerical ranges by endpoints includes all numbers and fractions subsumed within the respective ranges, as well as the recited endpoints. Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the disclosure described herein are capable of operation in other sequences than described or illustrated herein.

Reference in this specification may be made to devices, structures, systems, or methods that provide "improved" performance (*e.g*. increased or decreased results, depending on the context). It is to be understood that unless otherwise stated, such "improvement" is a measure of a benefit obtained based on a comparison to devices, structures, systems or methods in the prior art. Furthermore, it is to be understood that the degree of improved performance may vary between disclosed embodiments and that no equality or consistency in the amount, degree, or realization of improved performance is to be assumed as universally applicable.

### EXAMPLES

Examples of the implementation of the technology according to the present invention is given hereinbelow. The provision of examples is intended to assist the reader in understanding the technological concepts more easily, but it is not meant to identify the most important or essential features thereof, nor is it meant to limit the scope of the present invention.

As an example, a process will be described to sterilise medical material, using sterilization system of the herein described invention, consisting of medical a cylindrical multimode applicator operating at a f = 2.45 GHz that can be pressurized up to 12 bar and has an adjustable MW power up to 6 kW. To simulate liquid medical material, 10 dm³ tap water is placed inside a polypropylene box having a lid which is then heated by MW energy.

In the middle of the lid, a hole for monitoring the temperature of the liquid was foreseen such that an IR temperature sensor can constantly monitor the increase and/or decrease of the actual temperature of the liquid. Due to the fact that pressure is applied inside the sterilization system , the temperature measurement by the IR temperature sensor was performed through a glass window installed in the top of the microwave cavity of the sterilization system. To reduce the possibility of condensation onto this glass window which could potentially disturb the temperature measurements, the container was covered with a thin plastic bag made out of LDPE. Due to the fact that the temperature measurements are measured using an IR temperature sensor through the glass window and the transparent bag, an error will be present between the actual temperature of the liquid in the container, and the measured temperature by the IR temperature sensor.

To ensure that the minimum temperature is reached, while making sure that the maximum temperature is not exceeded, an error margin can be therefore input in the controller, taking into account this error in temperature measurement. In addition to reduce the risk of condensation due to the cold surface of the glass window, and thus further reduce the error margin of the IR temperature sensor, the glass window is pre-heated with hot air before starting the magnetron. To estimate the error in the measured IR temperature due to the glass window, some condensation and, especially, due to the plastic bag, the temperature of the liquid is measured before and after the MW heating with a temperature sensor PT100 and compared with the monitored value by the IR temperature sensor. As mentioned before, the error value can be entered in the controller such that the controller will correctly operate the sterilisation process. In the present example, the error margin was an increase of 15 °C when the liquid was measured with the IR temperature sensor through the bag and glass window, compared to the direct measurement using the PT100 sensor.

The applied overpressure during heating was 2.5 bara (3.5 bar total pressure), which corresponds to a boiling point of 138.9 °C for water. When sterilising, it is the aim to heat the liquid above 121°C to actually perform the sterilisation process, but to keep the temperature below the boiling point of the liquid.

The efficiency of the sterilisation step, meaning after applying the adequate temperature and dwell time, is evaluated by using Sterikon ^{®} Plus bioindicators (3 ampuls placed at various locations of the liquid). In theory, the required sterilization time at 121°C is 30 minutes. Sterikon ^{®} Plus bioindicators are designed for sterilisation processes at 121°C. Before revealing the result, which can be detected by the presence or absence of a change in colour, the indicators need to be incubated at 60°C for at least 24 hours. If the sterilisation has not been complete, the microbes in the liquid of the ampuls start to grow and cause a change in the colour so that the liquid turns from pink to yellow. If the sterilisation however was successful, no change in colour will be noticeable. In order to exclude the effects of post-handling and incubation conditions, the test batch of Sterikon ^{®} Plus bioindicators contains an untreated (non-sterilised but incubated) ampul as a reference.

A MW power of 4 kW (highest power without reflection) was applied until a product temperature of above 150°C was measured by the IR temperature sensor, thus accounting for the error margin due to the measurement by the IR temperature sensor. After this temperature was reached, the power can be reduced to 1-3 kW to maintain the temperature at level 150-160°C by controlling the MW power and thus the temperature. The heating time at the aimed temperature was around 29 minutes.

After completion of the sterilisation process, the liquid was allowed to cool down to around 100°C before opening the pressure vessel. This temperature measurement was again performed by using the IR temperature sensor. The cooling down time was around 1.5 hours. In order to perform a control of the error value of the temperature measurement, the temperature of the liquid was measured by a temperature Sensor PT100 which was immersed in the liquid. The temperature measured by the PT100 sensor was approximately 85°C, resulting in a difference in measurement of about 15°C between the IR measurement through the plastic bag and the actual temperature of the liquid inside the container and bag.

As an additional control measure, the colour of the bioindicators indicated that the sterilization cycle had been successful due to the fact that the colour of the liquid in the ampuls remained pink during incubation. Hence, it can be concluded that heating the pressurized air inside the container and bag to 150 °C when measured by the IR temperature sensor and maintaining this temperature during a time period of about 30 minutes, will result in a successful sterilisation of the liquid in the container.

## Claims

1. Method (10) for treatment of a medical material (230), comprising the steps of:
- placing (20) the medical material (230) in a sealable container (210) comprised of a sterilisation-resistant and non-microwave absorbent material;
- placing (30) the container (210) in a pressure vessel (240);
- applying (50) microwaves (255) to the container (210) in the pressure vessel (240) to heat and sterilise or decontaminate the medical material (230); and,
- applying (40) overpressure to the container (210) during the microwave treatment to prevent deformation of the container (210).

2. The method (10) according to claim 1, wherein the heating rate is controlled to maintain a temperature within the container (210) during microwave treatment that is suitable for sterilising the medical material (230), wherein the temperature is preferably at least 121°C.

3. The method (10) according to any one of the preceding claims, wherein the heating rate is controlled to maintain the temperature within the container (210) below the boiling point of water during microwave treatment, preferably below 138.9°C at 2.5 bara.

4. The method (10) according to any one of the preceding claims, wherein the sealable container (210) comprises a valve configured to regulate pressure differences that can cause deformation of the sealable container (210).

5. The method (10) according to any one of the preceding claims, wherein the material of the container (210) comprises polypropylene, preferably consists of polypropylene, glass, ceramic or plastics.

6. The method (10) according to any one of the preceding claims, wherein the treatment process is **characterised by** at least one of the of the following parameters:
- a pressure, in the pressure vessel (240), between at least 1 bara and at most 12 bara, preferably between 1.5 bara and 2.5 bara, more preferably about 2 bara;
- a electric/magnetic field strength between at least 1 kV/m and at most 7 kV/m, preferably between 1.5 kV/m and 5 kV/m, more preferably about 3 kV/m;
- a microwave frequency between at least0.1 GHz to at most 6.0 GHz, preferably about 2.5 GHz;
- a treatment duration during which the microwaves (255) are applied for at least 10 minutes and at most 30 minutes, preferably between 15 minutes to 25 minutes.

7. The method (10) according to any one of the preceding claims, comprising the step of measuring (60) the temperature within the container (210) during microwave treatment, and controlling the power of the microwaves (255) applied to the container (210) based on the measured temperature to maintain a desired temperature or temperature range within the container (210); preferably wherein the temperature is measured optically with an infrared sensor (290).

8. The method (10) according to any one of the preceding claims, comprising the step of measuring the pressure within the container (210) during microwave treatment, and controlling the overpressure applied to the container (210) based on the measured pressure to maintain a desired pressure or pressure range within the container (210).

9. The method (10) according to any one of the preceding claims, comprising the step of placing the container in a transparent bag (225) adapted to prevent water condensation.

10. The method (10) according to any one of the preceding claims, wherein at least one sterilisation or decontamination indicator means is disposed in the container (210) for monitoring the efficacy of the treatment process; preferably wherein the indicator means comprises a bioindicator comprising a biological medium and a pH indicator configured to visually indicate that the biological medium is effectively sterilised or decontaminated.

11. The method (10) according to any one of the preceding claims, comprising the step of cooling (90) the container (210) with a cooling medium after microwave treatment, preferably by applying a cooling fluid to the container (210).

12. System (200) for treatment of medical material (230), comprising
- a container (210) comprising a sterilisation-resistant and non-microwave absorbent material, and a lid (220) forming a seal with the container (210); wherein the container (210) is adapted to receive the medical material (230) and optionally an amount of water;
- a pressure vessel (240) adapted to receive the container (210) and apply pressure to it;
- at least one microwave source (250) adapted to generate microwaves (255) in the pressure vessel (240);
- a controller (260) operatively connected to the pressure vessel (240) and at least one microwave source (250), wherein the controller (260) is configured to apply microwaves (255) to the container (210) to heat and sterilise or decontaminate the medical material (230) while applying overpressure to the container (210) during microwave treatment to prevent deformation.

13. The system (200) according to claim 12 wherein the sealable container (210) comprises a valve configured to regulate pressure differences that can cause deformation of the sealable container (210).

14. The system (200) according any one of the claims 12 or13, comprising a temperature sensor (290) configured for measuring the temperature in the container (210) and is in communication with the controller (260), wherein the controller (260) is configured to control the power of the microwaves (255) applied to the container (210) based on the measured temperature to reach and/or maintain a desired temperature or temperature range within the container (210).

15. The system (200) according to any one of the claims12 to 14, wherein the pressure vessel (240) comprises a sight window (245), preferably formed in a wall of the pressure vessel (240), that allows visual inspection of the container (210); and an infrared temperature sensor (290), configured to measure the temperature of the container (210) through the sight window (245) during microwave treatment; wherein the sight window (245) is adapted to prevent condensation, preferably with a heating means configured to warm the sight window (245).
